# EUROPEAN PATENT APPLICATION

(11) **EP 4 082 451 A1**
(43) Date of publication of application: **02.11.2022**
(21) Application number: 20908043.1
(22) Date of filing: 04.09.2020
(51) Int. Cl.: A61B 17/12

(54) **OCCLUDER AND SYSTEM**

(30) Priority: 24.12.2019 CN 201911349235
(71) Applicant: Shanghai MicroPort Endovascular MedTech (Group) Co., Ltd., Shanghai 201318 (CN)
(72) Inventor: TU, Chunlin, Shanghai 201318 (CN); LU, Qingsheng, Shanghai 201318 (CN); ZHANG, Guangjian, Shanghai 201318 (CN); ZHANG, Zhaoduo, Shanghai 201318 (CN); YUAN, Zhenyu, Shanghai 201318 (CN); ZHU, Qing, Shanghai 201318 (CN)
(74) Representative: Patentship Patentanwaltsgesellschaft mbH
(86) International application number: PCT/CN2020/113613
(87) International publication number: WO 2021/128938

(57) **Abstract**

An occluder (100) and system. The occluder (100) includes a body (110) including a shaping wire (111) and a spring coil (112), the shaping wire (111) formed by a resilient metal wire and configured to have a predetermined shape, the spring coil (112) sleeved on the shaping wire (111) in order to shape the body (110) with the predetermined shape. Through maintaining the shape of the body (110) by means of the shaping wire (111), the body (110) is able to effectively block the breach (30) in aortic dissection treatments.

## Description

### TECHNICAL FIELD

The present application relates to the field of medical devices and, in particular, to an occluder and an occluding system.

### BACKGROUND

Aortic dissection (AD) is a condition in which blood flowing in the aortic lumen enters the aortic media from a breach in the aortic intima, and separates expands the tissue of the media apart along the longitudinal direction of the aorta, creating two separated true and false lumens in the aortic wall. AD is an acute, life-threatening cardiovascular disease featuring an abrupt onset, rapid progression and a very high mortality rate. In the current clinical practice, endovascular aortic repair (EVAR) is the most widely used AD therapy. In an EVAR procedure, an implant is delivered into the aortic lumen to occlude primary breach in the aortic lumen so that blood flowing in the false lumen will slow down or even stop, leading to the formation of a thrombus. With the thrombus being gradually organized and absorbed, the true lumen will restore its normal anatomy state and function to supply blood to the visceral arteries, and the aorta is finally reconstructed. Compared with other therapies (e.g., open surgery), EVAR is advantageous in less trauma, lower perioperative mortality and faster recovery.

Theoretically, once blood inflow to the false lumen disappears following the occlusion of the primary breach in the aortic true lumen, the pressure in the false lumen suddenly drops, which subsequently relieves the compression to the true lumen. As a result, recovery of the true lumen's normal anatomy will be realized over time as the false lumen gradually undergoes thrombosis and organization of the thrombosis in the false lumen. However, in practice, unsatisfactory aortic reconstruction has been observed in some AD patients who received EVAR, mainly for the reason that EVAR could occlude only the primary breach near the heart. Whereas, most AD patients often have multiple breaches and most of the multiple breaches are located around the visceral arteries (i.e., the breach being the distal breach away from the heart). At present, such distal breaches are treated mainly by (1) occlusion with additional implants, (2) aortic replacement or (3) conservative management with intensive surveillance. Approach (1) may lead to insufficient visceral blood supply that can endanger the patient's life, while approach (2) is associated with the risk of high postoperative mortality and paraplegia rates. Approach (3) may lead to insufficient postoperative expansion of the distal aortic true lumen and continuous false lumen perfusion, which may seriously affect benign reconstruction of the distal aorta and the patient's late prognosis.

For AD, the desirable surgical approach is to repair breaches for total aorta and facilitate false lumen thrombosis. At present, distal breaches are commonly occluded by occluders. As a "targeted therapy" in the field of endovascular treatment, an occluder aims for isolation of a breach without affecting blood supply to adjacent important branch arteries, which can minimize the likelihood of postoperative ischemia of important branch arteries or organ infarction. Therefore, the occluder has many advantages in AD treatment, which brings a broad clinical application value to the occluder. However, existing occluders for occluding breaches are associated with various problems. For example, the existing occluder has a slack shape after released, leading to a poor occlusion. Moreover, the existing occluder lacks anchoring means used for its fixation in a blood vessel, which results in the occlusion failure due to displacement under the impact of blood flow.

### SUMMARY

It is an object of the present application to provide an occluder and an occluding system that are capable of maintaining a predetermined configuration after released, which is able to ensure good occlusion, facilitate false lumen thrombosis and achieve true lumen reconstruction.

To this end, the present application provides an occluder comprising a body comprising a shaping wire and a spring coil, the shaping wire formed by a resilient metal wire and configured to have a predetermined shape, the spring coil sleeved over the shaping wire in order to shape the body with the predetermined shape.

Optionally, the occluder further comprises an anchoring mechanism connected to one end of the shaping wire. The anchoring mechanism is rotatable about an axis of the shaping wire and configured to secure the body onto an object.

Optionally, the shaping wire is coiled into a continuous, curved structure to form the predetermined shape.

Optionally, the predetermined shape is a helical shape, and the shaping wire is helically coiled along a certain axis to form the helical shape.

Optionally, the predetermined shape is a circular cone. The shaping wire is coiled into a plurality of arc-shaped sections connected sequentially. The plurality of arc-shaped sections are sequentially arranged in an axial direction of the circular cone and adjacent arc-shaped sections are eccentrically arranged.

Optionally, the body has a first end and a second end opposite the first end in the axial direction of the circular cone. Along a direction from the first end to the second end, the circular cone has an increased radial cross section and each of the plurality of arc-shaped sections has an increased radial size. The anchoring mechanism is provided at the second end.

Optionally, the predetermined shape is a spherical or hexahedral shape.

Optionally, the anchoring mechanism comprises a positioning portion configured to connect with the object, the positioning portion being a barb or a positioning disc

Optionally, the positioning portion is provided with a sharp end configured to pierce into an inner wall of a blood vessel to secure the body.

Optionally, the occluder further comprises thrombosis-enhancing villi attached to the body.

Optionally, the thrombosis-enhancing villi are wounded around the shaping wire and clamped and secured by the spring coil.

Optionally, at least two thrombosis-enhancing villi are arranged on the shaping wire and spaced apart from one another, and a distance between two adjacent thrombosis-enhancing villi along an axis of the shaping wire is in a range of from 5 mm to 10 mm.

Optionally, each of the thrombosis-enhancing villi has a length in a range of from 15 mm to 25 mm.

Optionally, the spring coil has an inner diameter ranging from 0.5 mm to 1.0 mm.

To achieve the above objective, the present application also provides an occluding system comprising a stent and at least one occluder as defined above. The occluder is coupled to the stent.

Compared with the prior art, the occluder and occluding system of the present application offer the following advantages:
First, the occluder comprises a body comprising a shaping wire and a spring coil, the shaping wire formed by a resilient metal wire and configured to have a predetermined shape, the spring coil sleeved over the shaping wire in order to shape the body with the predetermined shape. Through maintaining the shape of the body by means of the shaping wire, the body is able to effectively block the breach.
Second, the occluder also comprises the anchoring mechanism made of a resilient metal and provided on the body, the anchoring mechanism configured to secure the body onto an object. The anchoring mechanism enables to fix the position of the body so as to avoid displacement of the body.
Third, the anchoring mechanism is coupled to one end of the shaping wire and is rotatable about the axis of the shaping wire. In this way, the torsional effect generated by the spring coil during delivery and release of the occluder in an aorta is able to be eliminated.

### BRIEF DESCRIPTION OF THE DRAWINGS

Fig. 1 is a section view of a patient's aorta with aortic dissection (AD).
Fig. 2 is a structural schematic of an occluder according to embodiment one of the present application, in which a ball head is not shown.
Fig. 3 schematically illustrates an occlusion system according to embodiment one of the present application implanted into an aorta to occlude the breach therein, in which only one occluder is shown.
Fig. 4 is a structural schematic of a shaping wire in the occluder according to embodiment one of the present application.
Fig. 5 is a structural schematic of a spring coil in the occluder according to embodiment one of the present application, in which the spring coil is not sleeved over the shaping wire yet.
Fig. 6 is a structural schematic of a body in the occluder according to embodiment one of the present application.
Fig. 7 is a structural schematic of the occluder according to embodiment one of the present application, in which the occluder is stretched to a straightened configuration and an anchoring mechanism is not shown.
Fig. 8a is a structural schematic of an anchoring mechanism in the occluder according to embodiment one of the present application.
Fig. 8b is a structural schematic of a variant of the anchoring mechanism in the occluder of Fig. 8a.
Fig. 8c is a structural schematic of another variant of the anchoring mechanism in the occluder of Fig. 8a.
Fig. 8d is a structural schematic of yet another variant of the anchoring mechanism in the occluder of Fig. 8a.
Fig. 9 schematically illustrates how the anchoring mechanism in the occluder according to embodiment one of the present application is coupled to the shaping wire.
Fig. 10 schematically illustrates how the anchoring mechanism in the occluder according to embodiment one of the present application is coupled to a stent.
Fig. 11a schematically illustrates the connection between the occluder according to embodiment one of the present application and the delivery system.
Fig. 11b schematically illustrates another connection between the occluder according to embodiment one of the present application and the delivery system.
Fig. 12 is a structural schematic of an occluder according to embodiment two of the present application.
Fig. 13 schematically illustrates an occlusion system according to embodiment two of the present application implanted in an aorta to occlude the breach therein, in which only one occluder is shown.
Fig. 14 is a structural schematic of an occluder according to embodiment three of the present application.
Fig. 15 schematically illustrates an occlusion system according to embodiment three of the present application implanted in an aorta to occlude the breach therein, in which only one occluder is shown.
Fig. 16a is a structural schematic of an occluder according to embodiment four of the present application, as seen in one direction.
Fig. 16b is a structural schematic of the occluder of Fig. 16a, as seen in another direction.
Fig. 16c is a structural schematic of the occluder of Fig. 16a, as seen in yet another direction.
Fig. 17 schematically illustrates an occlusion system according to embodiment four of the present application implanted in an aorta to occlude the breach therein, in which only one occluder is shown.
Fig. 18 is a structural schematic of an occluder according to embodiment five of the present application.
Fig. 19 schematically illustrates an occlusion system according to embodiment five of the present application implanted in an aorta to occlude the breach therein, in which only one occluder is shown.

In the Figures:
- 100: Occluder;
- 110: Body;
- 111: Shaping Wire, 112 Spring Coil, 113 Ball Head;
- 120: Anchoring Mechanism;
- 121: Anchor, 122 Sharp End, 123 Sleeve, 124 First Limiting Member;
- 130: Thrombosis-Enhancing villus;
- 200: Stent;
- 210: hollowed-out structure;

- 300: Importing Device;
- 310: Detaching Mechanism;
- 10: True Lumen, 20 False Lumen, 30 Breach.

### DETAILED DESCRIPTION

Objects, advantages and features of the present application will become apparent upon reading the following detailed description with reference to the accompanying drawings. It should be noted that the drawings are provided in a very simplified form not necessarily drawn to scale, for the only purpose to facilitate convenient and explicit description of embodiments of the present application.

As used herein, the singular terms "a," "an" and "the" include their plural referents, while the plural form such as the term "plurality" means two or more, unless the context clearly dictates otherwise. As used herein, the term "or" is generally employed in the sense including "and/or", unless the context clearly dictates otherwise, and the terms "attach", "couple" and "connect" in their various forms should be interpreted in a broad sense, which can for example refer to a fixed connection, a detachable connection, or an integral connection, or to a mechanical connection or an electrical connection, or to a direct connection or an indirect connection with one or more elements interposed between the connected ones, or to mutual communication between the interiors of two elements or interaction between two elements. A person of ordinary skilled in the art would be able to understand the specific meanings of these terms in this context based upon specific situations. In the figures, identical or similar reference numerals will be used to identify identical or similar elements.

As used herein, "proximal end" or "distal end" refers to the relative orientation, relative position, or direction of elements or actions that are relative to each other from the perspective of an operator operating the device. Yet without wishing to be limiting in any sense, the "proximal end" generally refers to the end of the medical device close to the operator during its normal operation, while the "distal end" generally refers to the end that enters into the body of the patient first.

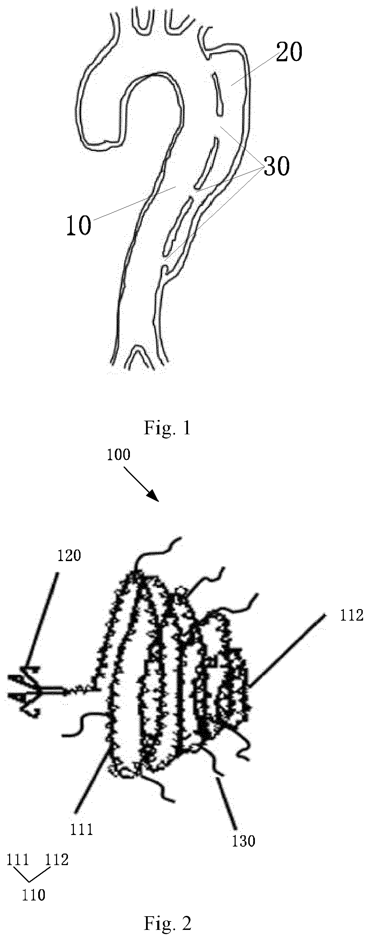

Fig. 1 is a section view of a patient's aorta with aortic dissection (AD). As shown in Fig. 1, when an AD is occurred in a patient, the aortic intima is torn and separated from the aortic media, which lead to the formation of a true lumen 10 and a false lumen 20 that communicate with each other via the breaches 30. It is an object of embodiments of the present application to provide a occluder that capable of filling the false lumen 20 and closing the breaches 30 to block blood inflow to the false lumen 20 and facilitate thrombosis of the false lumen 20 in AD treatments.

Fig. 2 is a structural schematic of an occluder 100 according to embodiment one of the present application. As shown in Fig. 2, the occluder 100 includes a body 110 comprising a shaping wire 111 and a spring coil 112. The shaping wire 111 is a resilient metal wire configured to have a predetermined shape, and the spring coil 112 is sleeved over the shaping wire 111.

During AD treatment, the body 110 is filled in the false lumen 20 and occludes a breach 30. By employing the resilient metal wire as the shaping wire 111 that is configured to have a predetermined shape, the body 110 also presents the said predetermined shape as the spring coil 112 is sleeved over the shaping wire 111. The body 110 is deformed during the delivery of the occluder 100 into a blood vessel, while returning back to the predetermined shape under the resilience force from the shaping wire 111 after the release of the occlude 100 into the blood vessel. In other words, embodiments of present application maintain the shape of the body 110 by means of the shaping wire 111so as to effectively occlude the breach 30.

The occluder 100 further includes an anchoring mechanism 120. The anchoring mechanism 120 is made of a resilient metal. The anchoring mechanism 120 is disposed on the body 110 and connected to one end of the shaping wire. The anchoring mechanism is rotatable about an axis of the shaping wire and configured to secure the body 110 to an object.

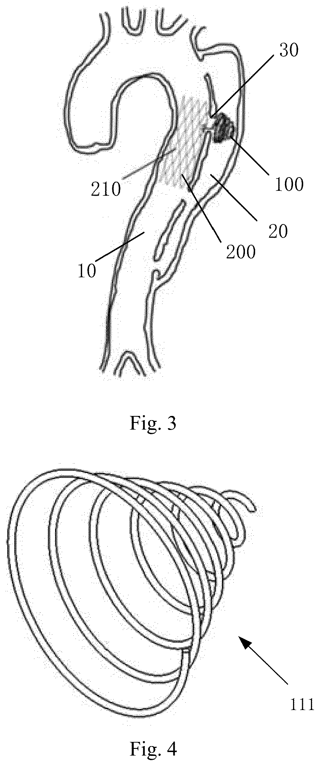

With particular references to Fig. 3, the occluder 100 can be used with a stent 200 in AD treatment. As shown in Fig. 3, when the occluder 100 is used with the stent 200 to treat AD, the stent 200 is secured in and supports the true lumen 10 in order to relieve compression of the true lumen from the false lumen. The occluder 100 is implanted in the false lumen 20 and positioned at the breach 30 in order to reduce or even block the blood inflow to the false lumen 20 to facilitate thrombosis of the false lumen 20. The occluder includes the anchoring mechanism 120, through which the occluder 100 is be coupled to the stent 200. In particular, the stent 200 have a hollow-out structure 210, and the anchoring mechanism 120 can be inserted in the hollow-out structure 210 and thus coupled to the stent 200 (i.e., the stent being the aforementioned object), so that the occluder 100 is retained at the breach 30 to achieve the positioning of the body 110, preventing the body 110 from moving away from the breach 30 under the impact of blood flow to further affect the treatment effect.

The specific structure and fabrication process of the occluder 100 according to this embodiment will be described below.

The shaping wire 111 can be made of a shape memory alloy (e.g., nickel-titanium alloy, copper-nickel alloy, copper-zinc alloy or other alloy), a cobalt-chromium alloy, stainless steel or the like. The shaping wire 111 is coiled to form the predetermined shape and then subjected to the heat treatment in order to enable the shaping wire 111 to present the predetermined shape in its natural state. The shaping wire 111 deforms when an external force is applied thereto and returns back to the predetermined shape once the external force is removed.

Preferably, the shaping wire 111 is coiled to form a continuous, smooth curvilinear structure, so that the eventually formed body 110 does not have any sharp corner, which may avoid injuries to the wall of a blood vessel to result in secondary damages during implantation of the body 110.

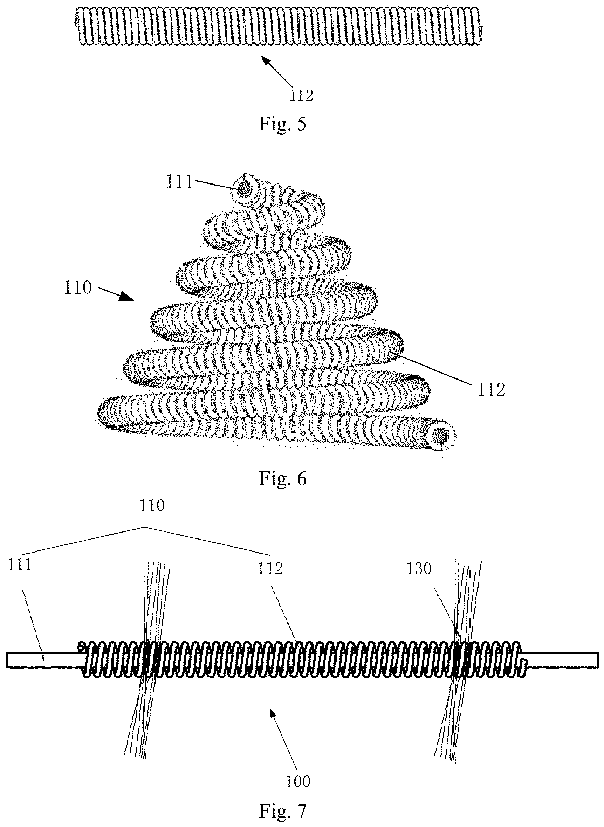

As shown in Fig. 5, the spring coil 112 may be fabricated by coiling a metal wire around a core shaft to form a spring shape and then thermally treating both ends of the metal wire to retain the spring shape. The spring coil 112 may be made up of a nickel-titanium wire, a platinum-tungsten wire, a stainless steel wire or the like. Depending on the actual needs, the core shaft may have an outer diameter in the range of from 0.5 mm to 1.0 mm so that the spring coil 112 has an inner diameter in the range of from 0.5 mm to 1.0 mm.

After that, a force is applied to the shaping wire 111 to straighten the shaping wire 111 from its predetermined shape, and the spring coil 112 is then sleeved over the shaping wire 111. Once removing the force, the shaping wire 111 returns back to the predetermined shape by virtue of its own resilience. In this way, the body 110 having the predetermined shape can be obtained (as shown in Fig. 6). Preferably, in order to prevent uncoiling of the spring coil 112 during the release of the occluder 100, opposing ends of the spring coil 112 are connected to opposing ends of the shaping wire 111 respectively.

Further, in this embodiment, the predetermined shape is a helix. For example, with combined reference to Figs. 2 and 4, the shaping wire 111 is helically coiled along an axis to form the helix having a conical shape. In this way, along the extending direction of the axis, the body 110 has a first end and a second end opposing the first end and has a radial cross section gradually increasing from the first end to the second end. The anchoring mechanism 120 is provided at the second end of the body 110, and a ball head (not shown in Figs. 2 and 4) is arranged at the first end thereof. In an alternative implementation, the shaping wire can be helically coiled along an axis to form the helix having a cylindrical shape (not shown).

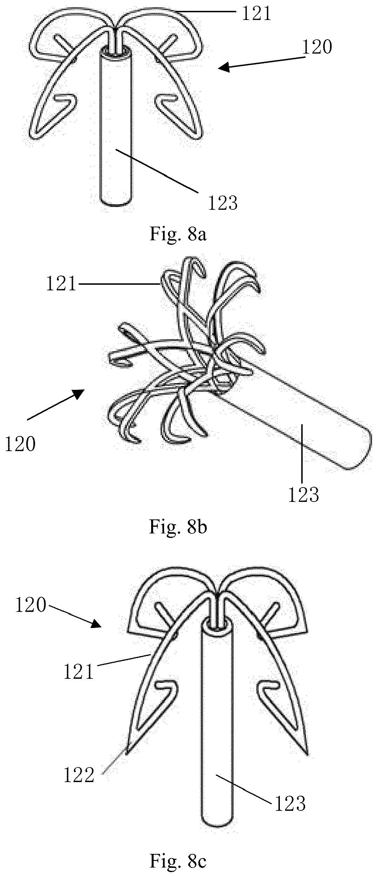

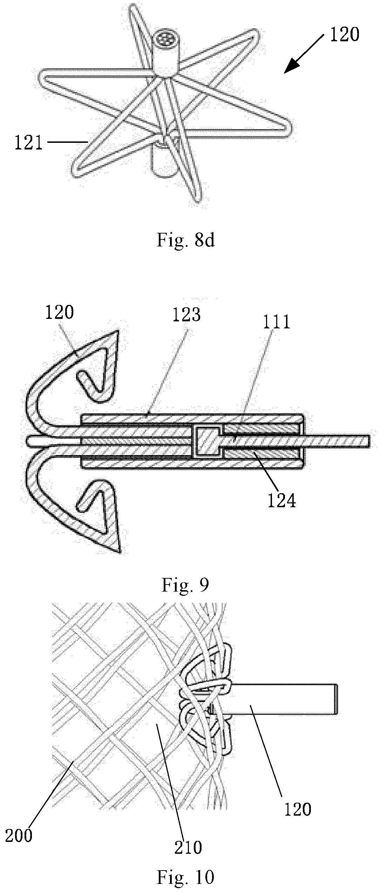

The anchoring mechanism 120 may be made of a shape memory alloy (e.g., a nickel-titanium, copper-nickel, copper-zinc or other alloy), a cobalt-chromium alloy, stainless steel or the like. As shown in Figs. 8a to 8d, the anchoring mechanism 120 has a positioning portion 121. In some embodiments, the positioning portion 121 is a barb, as shown in Fig. 8a, 8b and 8c. In alternatively embodiments, the positioning portion 121 is a positioning disc constructed by sequentially arranging multiple struts that are bended into V shapes around an axis (as shown in Fig. 8d). The anchoring mechanism 120 may also have any other suitable structure that allows its coupling to the stent 200, and the present application put no limitation to this.

Further, as shown in Fig. 8c, the positioning portion 121 has sharp ends. The sharp ends are configured to pierce into an inner wall of a blood vessel to secure the occluder 100 at the breach 30 when the connection between the positioning portion 121 and the stent 200 is unable to locate the occluder 100 at the breach 30.

The anchoring mechanism 120 is subjected to a significant force during the implantation of occluder 100 into the blood vessel. In order to avoid the anchoring mechanism 120 from transmitting the force to the spring coil 112 to cause detachment of the spring coil 112 from the shaping wire 111, the anchoring mechanism 120 is preferred to be connected to the shaping wire 111 and is able to rotate about the axis of the shaping wire 111.

With particular reference to Fig. 9, the anchoring mechanism 120 also includes a sleeve 123 to which the positioning portion 121 is fixed. The sleeve 123 has a first inner cavity. One end portion of the shaping wire 111 is inserted in the first inner cavity of the sleeve 123. In addition, a first limiting member 124 is provided in the first inner cavity, while the end portion of the shaping wire 111 is provided with a second limiting member (not shown). The second limiting member is engaged with the first limiting member to keep the shaping wire 111 still relative to the sleeve 123 in the axial direction while allowing the shaping wire 111 to rotate relative to the sleeve 123 in the circumferential direction. The advantageous of this arrangement is describled below. With reference to Figs. 3 and 10, insertion of the anchoring mechanism 120 into the hollow-out structure 210 is required to realize coupling between the anchoring mechanism 120 and the stent 200 after the occluder has been implanted into the blood vessel. However, the shaping wire 111 is straightened during the delivery of the occluder 100. After the release of occluder 100 in the blood vessel, the shaping wire 111 return back to its predetermined shape under the action of its own resilience. In this recovery process, the shaping wire 111 twists. In this case, the relative rotation between the sleeve 123 and the shaping wire 111 can be utilized to offset the adverse effect of the twisting of the shaping wire 111 on the posture of the anchoring mechanism 120, so that the anchoring mechanism 120 is able to enter into the hollow-out structure 210 of stent 210 smoothly.

Further, as shown in Figs. 2 and 7, the occluder 100 further includes thrombosis-enhancing villi 130, which are attached to the body 110 to expand the filling volume of the occluder 100 for facilitating thrombosis in the false lumen 20. The thrombosis-enhancing villi 130 are made of at least one polymer selected from the group consisting of polyethylene terephthalate (PET), polyamide (PA), polyethylene (PE), polypropylene (PP), polyurethane (PU), and polylactic acid (PLA). These polymeric materials are made into 300-700 D fully drawn yarns or stretch textured yarns, which are then wound around the shaping wire 111 two to three turns and clamped by two adjacent turns of the spring coil 112 to form the thrombosis-enhancing villi 130 (as shown in Fig. 7). Additionally, in the body 110, the thrombosis-enhancing villi 130 may be arranged in the axial direction of shaping wire 111 and spaced apart from one another. The distance between two adjacent thrombosis-enhancing villi along an axis of the shaping wire is in a range of from 5 mm to 10 mm. Further, each of the thrombosis-enhancing villi 130 has a length (i.e., the length from the connection of the thrombosis-enhancing villus to the shaping wire 111 to the free end of the thrombosis-enhancing villus) in the range of from 15 mm to 25 mm.

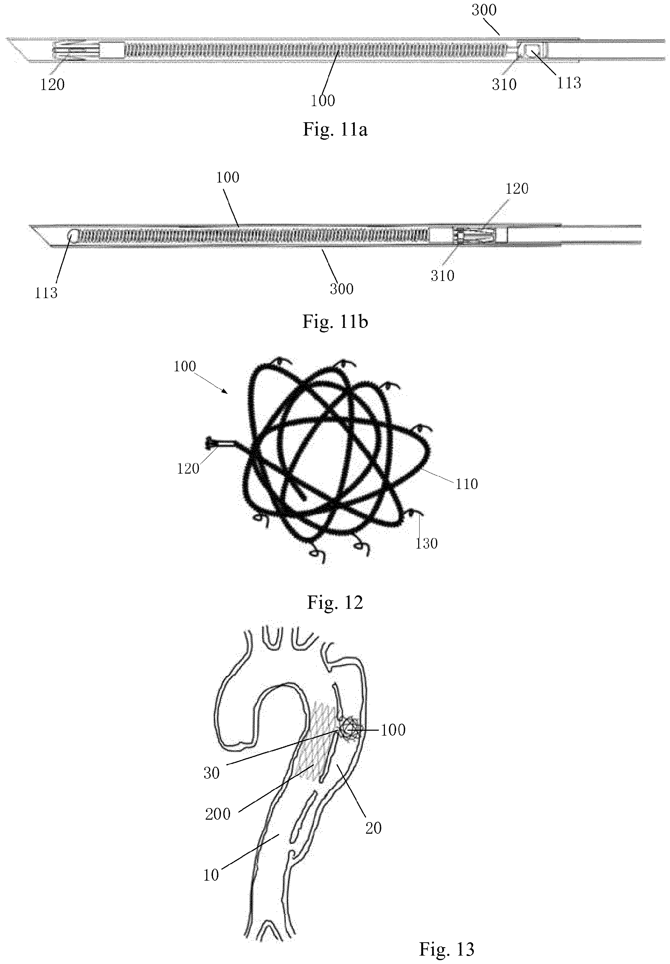

Referring to Fig. 11a, during AD treatment, the occluder 100 is delivered into the blood vessel via an importing device 300. The importing device 300 has a second inner cavity for receiving the occluder 100.The second inner cavity extends along an axis of the importing device 300 and has a proximal end and a distal end opposing the proximal end. At the distal end, there is provided a detaching mechanism 310. In order to deliver the occluder 100 into the blood vessel, the occluder 100 is straightened and loaded in the second inner cavity. In some embodiments, the ball head 113 at the first end of the occluder 100 is coupled to the detaching mechanism 310. In this case, the occluder 100 is delivered into the false lumen 20 from the true lumen 10. Upon the occluder 100 reaching the target site, the delivery system 300 releases the occluder 100 and disconnects the connection between the detaching mechanism 310 and the ball head 113. Alternatively, as shown in Fig. 11b, in some other embodiments, the anchoring mechanism 120 in the occluder 100 is coupled to the detaching mechanism 310. In this case, the occluder 100 is delivered into the breach 30 through the false lumen 20.

Fig. 12 is a structural schematic of an occluder 100 according to embodiment two of the present application. In the following description, only differences between embodiment two and embodiment one will be explained, and a description of any common feature between them will be omitted for the sake of brevity.

As shown in Fig. 12, in embodiment two, the shaping wire 111 is coiled into an outer contour of spherical structure (the spherical structure here including the spherical structure in geometry and the roughly spherical structure). The shaping wire 111 has its one end projecting out of the spherical structure to facilitate connection with the anchoring mechanism 120.

As shown in Fig. 13, the occluder 100 according to embodiment two may also be used with a stent 200 in AD treatment.

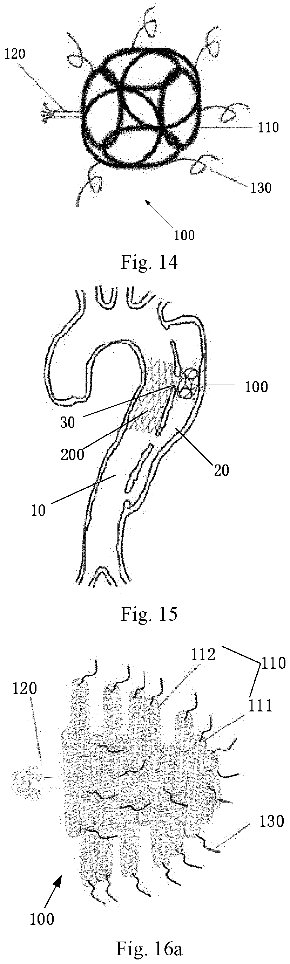

Fig. 14 is a structural schematic of an occluder 100 according to embodiment three of the present application. As shown in Fig. 14, in embodiment three, the shaping wire 111 is coiled into an outer contour of a regular hexahedral structure. As shown in Fig. 15, the occluder 100 according to embodiment three may also be used with a stent 200 in AD treatment.

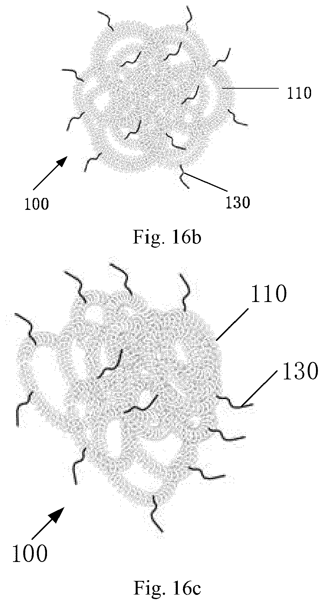

Figs. 16a to 16c are structural schematics of an occluder 100 according to embodiment four of the present application. As shown in Figs. 16a to 16c, the body 110 of the occluder 100 in embodiment four generally appears like a circular cone, which differs from embodiment one in that the shaping wire is twisted to form a plurality of arc-shaped sections connected sequentially. In this case, in an axial direction of the circular cone, the plurality of arc-shaped sections are sequentially arranged and adjacent arc-shaped sections are eccentrically arranged. This arrangement is advantageous in that there is no large void in the body 110, allowing better occlusion of the breach 30 by the occluder 100. In addition, these arc-shaped sections have increased radial sizes in the direction from the first end to the second end, so that the one of these arc-shaped sections having the smallest radial size is closest to the vessel wall. Further, each of the arc-shaped sections may be approximate to circle, and the smaller the radial size of an arc-shaped section is, the more difficult a corresponding portion of the spring coil 112 will be to deform during release of the occluder 100. This helps in improving the performance of the occluder 100 during use.

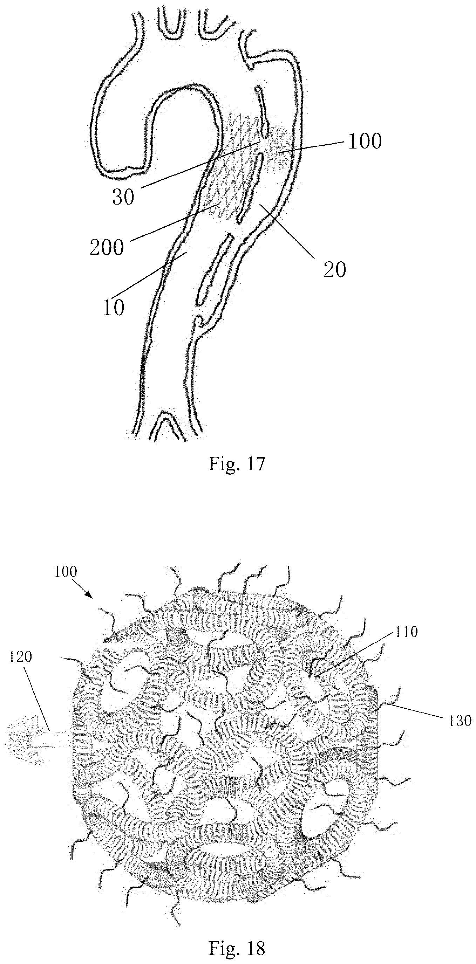

As shown in Fig. 17, the occluder 100 according to embodiment four may also be used with a stent 200 in AD treatment.

Fig. 18 is a structural schematic of an occluder 100 according to embodiment five of the present application. As shown in Fig. 18, the occluder 100 is generally a spherical structure. It differs from embodiment two in that one portion of the shaping wire 111 is coiled into the outer contour of the spherical structure and the rest portion of the shaping wire 111 is coiled and located inside the hollow chamber of the spherical structure. The shaping wire 111 has its one end projecting out of the spherical structure to facilitate connection with the anchoring mechanism 120.

The shaping wire 111is coiled to present a plurality of arc-shaped sections, which constitutes the spherical structure. Preferably, each of the arc-shaped sections may be approximately circular. In this embodiment, since the shaping wire 111 is coiled into the spherical structure and is also coiled inside the spherical structure, when the spring coil 112 is sleeved over the shaping wire 111 to form the body 110, there is no large void present in body 110, which helps in blocking blood flow. Moreover, during release of the shaping wire 111 that forms the spherical structure, even when the arc-shaped sections kinks, there will be sufficient room for the kinked sections to reverse and return back to the predetermined positions under the resilience of the shaping wire 111. Therefore, the overall structure of the body 110 will not be affected, and the occlusion performance of the occluder 100 will be ensured.

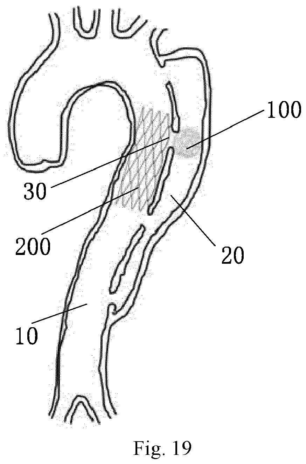

As shown in Fig. 19, the occluder 100 according to embodiment five may also be used with a stent 200 in AD treatment.

On the basis of the above described occluders 100, another object of the present application is to provide an occluding system for use in AD treatment. Referring to Figs. 3, 13, 15, 17 and 19, the occluding system includes the occluder 100 and a stent 200. The stent 200 is positioned in the true lumen 10 and has a hollow-out structure 210. The occluder 100 is implanted in the false lumen 20 and positioned at a breach 30, while the anchoring mechanism 120 on the occluder 100 is configured to be inserted into the hollow-out structure 210 for attachment to the stent 200.

Alternatively, the occluding system includes at least one occluder 100. In fact, a length of the stent 200 is preferred to cover all the breaches 30 in the blood vessel. The number of occluders 100 equals to the number of breaches 30, so that each breach 30 is provided with one occluder 100 to block the blood flow into the false lumen 20, facilitating thrombosis in the false lumen 20.

In the occluder 100 and occluding system provided in embodiments of the present application, the occluder 100 includes a body 110 and an anchoring mechanism 120. The body 110 includes a shaping wire 111 and a spring coil 112. The shaping wire 111 is formed by a resilient metal wire and configured to have a predetermined shape. The spring coil 112 is sleeved over the shaping wire 111 in order to shape the body with the predetermined shape. The anchoring mechanism 120 is provided on the body 110 and configured to be coupled to an object (the stent 200). By virtue of the resilience of the shaping wire 111, the body 110 is able to maintain the predetermined shape after the release of the body 110, ensuring occlusion of a breach 30. Moreover, the anchoring mechanism 120 is configured to retain the occluder 100 at the breach 30, avoiding the displacement of the body under the effect of blood flow, which would influence the occlusion effect.

Although the present application has been disclosed above, it is not limited to the above disclosure. Those skilled in the art can make various modifications and variations to the present application without departing from the spirit and scope thereof. Accordingly, the invention is intended to embrace all such modifications and variations if they fall within the scope of the appended claims and equivalents thereof.

## Claims

1. An occluder comprising a body, wherein the body comprises a shaping wire and a spring coil, the shaping wire formed by a resilient metal wire and configured to have a predetermined shape, the spring coil sleeved over the shaping wire in order to shape the body with the predetermined shape.

2. The occluder of claim 1, further comprising an anchoring mechanism connected to one end of the shaping wire, wherein the anchoring mechanism is rotatable about an axis of the shaping wire and configured to secure the body onto an object.

3. The occluder of claim 2, wherein the shaping wire is coiled into a continuous curved structure to form the predetermined shape.

4. The occluder of claim 3, wherein the predetermined shape is a helical shape, and the shaping wire is helically coiled along a certain axis to form the helical shape.

5. The occluder of claim 3, wherein the predetermined shape is a circular cone, wherein the shaping wire is coiled into a plurality of arc-shaped sections connected sequentially, and wherein the plurality of arc-shaped sections are sequentially arranged in an axial direction of the circular cone and adjacent arc-shaped sections are eccentrically arranged.

6. The occluder of claim 5, wherein the body has a first end and a second end opposite the first end in the axial direction of the circular cone, wherein, along a direction from the first end to the second end, the circular cone has an increased radial cross section and each of the plurality of arc-shaped sections has an increased radial size, and wherein the anchoring mechanism is provided at the second end.

7. The occluder of claim 3, wherein the predetermined shape is a spherical or hexahedral shape.

8. The occluder of claim 2, wherein the anchoring mechanism comprises a positioning portion configured to connect with the object, the positioning portion being a barb or a positioning disc.

9. The occluder of claim 8, wherein the positioning portion is provided with a sharp end configured to pierce into an inner wall of a blood vessel to secure the body.

10. The occluder of any one of claims 1 to 9, further comprising thrombosis-enhancing villi attached to the body.

11. The occluder of claim 10, wherein the thrombosis-enhancing villi are wounded around the shaping wire and clamped and secured by the spring coil.

12. The occluder of claim 11, wherein at least two thrombosis-enhancing villi are arranged on the shaping wire and spaced apart from one another, and wherein a distance between two adjacent thrombosis-enhancing villi along an axis of the shaping wire is in a range of from 5 mm to 10 mm.

13. The occluder of claim 12, wherein each of the thrombosis-enhancing villi has a length in a range of from 15 mm to 25 mm.

14. The occluder of any one of claims 1 to 9, wherein the spring coil has an inner diameter ranging from 0.5 mm to 1.0 mm.

15. An occluding system comprising a stent and at least one occluder according to any one of claims 1 to 14, the occluder being coupled to the stent.
